## Europäisches Patentamt

## European Patent Office

## Office européen des brevets

(19)

(11) Numéro de publication: **0 130 116 B1**

(12) **FASCICULE DE BREVET EUROPEEN**

(45) Date de publication du fascicule du brevet:
**10.12.86**

(21) Numéro de dépôt: **84401261.7**

(22) Date de dépôt: **19.06.84**

(51) Int. Cl.⁴: **C 12 M 1/00,** C 12 N 11/04, C 08 J 9/38

(54) **Paroi transparente en mousse de polyuréthane contenant des microorganismes, procédé pour sa préparation et utilisation de cette paroi dans un biophotoréacteur.**

(30) Priorité: **24.06.83 FR 8310489**

(43) Date de publication de la demande:
**02.01.85 Bulletin 85/1**

(45) Mention de la délivrance du brevet:
**10.12.86 Bulletin 86/50**

(84) Etats contractants désignés:
**BE DE GB IT**

(56) Documents cités:
**GB - A - 2 084 155**
**US - A - 2 732 663**
**US - A - 3 949 742**
**US - A - 4 250 267**

**SOL. ENERGY R & D EUR. COMMUNITY, vol. 1, Sec. D, 1982, pages 134-9, BE; M. BROUERS et al.: "Immobilization and stabilization of green and blue green algae in crosslinked serumalbumin glutaraldehyde and in polyurethane matrices"**

(73) Titulaire: **COMMISSARIAT A L'ENERGIE ATOMIQUE Etablissement de Caractère Scientifique Technique et Industriel, 31/33, rue de la Fédération, F-75015 Paris (FR)**

(72) Inventeur: **Adet, Bruno, 693, Avenue de Mazargues, F-13009 Marseille (FR)**
Inventeur: **Gudin, Claude, Traverse Sainte Anne, F-13100 Aix en Provence (FR)**
Inventeur: **Thepenier, Catherine, La Treille Muscate Route de Villelaure, F-84120 Pertuis (FR)**

(74) Mandataire: **Mongrédien, André et al, c/o BREVATOME 25, rue de Ponthieu, F-75008 Paris (FR)**

## Description

La présente invention a pour objet une paroi transparente en mousse de polyuréthane contenant des microorganismes, un procédé pour sa préparation et son utilisation dans un biophotoréacteur.

Depuis quelques années, on a développé de nouvelles techniques pour la culture des microorganismes capables de croître sous l'action de la lumière, en présence de gaz carbonique et d'un milieu de culture approprié en formant des produits ayant un intérêt économique dans l'alimentation, en pharmacie, ou pour la récupération assistée du pétrole.

En effet, certains microorganismes, par exemple la microrhodophycée Porphyridium Cruentum, peuvent excréter des polysaccharides sulfatés qui sont d'une grande valeur économique.

On peut aussi obtenir par culture de microorganismes tels que Botryococcus Braunii, des hydrocarbures, ce qui présente également un très grand intérêt.

Pour réaliser de telles cultures de microorganismes, il est nécessaire de mettre ceux-ci en contact avec un milieu liquide nutritif approprié et avec du gaz carbonique, et d'exposer simultanément le microorganisme à la lumière solaire ou à la lumière artificielle. On peut ensuite extraire du milieu liquide nutritif le produit synthétisé par le microorganisme. Ces cultures sont généralement réalisées dans des biophotoréacteurs qui sont conçus de manière à pouvoir assurer une irradiation des microorganismes par de la lumière solaire ou artificielle et une mise en contact des microorganismes avec un milieu approprié.

Un biophotoréacteur de ce type est décrit, par exemple, dans le brevet américain 3 955 317 publié le 11 mai 1976. Selon ce brevet, le biophotoréacteur est constitué par un système tubulaire transparent aux rayonnements solaires, contenant des microalgues en suspension dans un liquide nourricier au sein duquel est injecté du gaz carbonique. Les cellules végétales réalisent la photopolymérisation du gaz carbonique en un produit chimique désiré, par exemple en un produit à usage alimentaire ou pharmaceutique ou en un hydrocarbure, selon la nature de la microalgue utilisée, et cette réaction s'accompagne également d'un dégagement d'oxygène. Pour éviter une sédimentation prématurée de la biomasse cellulaire à l'intérieur du réacteur, il est nécessaire de la mettre en mouvement. Ceci peut être effectué en utilisant un système tubulaire ouvert à ses deux extrémités et en injectant en continu à l'entrée un mélange de liquide nourricier et de microalgues et en extrayant à la sortie le mélange. On sépare ensuite par centrifugation les microalgues et les métabolites fabriqués par celles-ci du milieu liquide.

L'utilisation d'un biophotoréacteur de ce type présente certains inconvénients car il nécessite une énergie importante, d'une part, pour la mise en circulation des microalgues et, d'autres part, pour la séparation des métabolites, ce qui augmente le prix de revient des produits ainsi fabriqués.

Afin de limiter l'énergie dépensée, on a envisagé d'immobiliser les microalgues dans une structure spongieuse de mousse de polyuréthane, comme cela est décrit dans le compte-rendu de l'Académie des Sciences du 6/7/1981 tome 293, série III, pages 35-37, intitulé «Production de polysaccharides sulfatés par un biophotoréacteur à cellules immobilisées de Porphyridium cruentum». Dans ce cas, on introduit les microorganismes au moment de la préparation de la mousse de polyuréthane en mélangeant par parties sensiblement égales les composés précurseurs de la mousse de polyuréthane et une suspension d'algues dans un liquide nourricier. La formation de la mousse et l'immobilisation des microalgues à l'intérieur de celle-ci s'effectuent simultanément en quelques dizaines de minutes à la température ambiante. On découpe ensuite la mousse en petits dés d'environ 1,5 cm de côté, puis on les introduit dans une colonne de verre qui contient un milieu liquide nutritif et on les expose au rayonnement solaire.

Les microalgues qui ont survécu à la polymérisation recolonisent alors tous les pores des dés de polyuréthanne qui sont irrigués en continu par le milieu liquide nutritif et par de l'air contenant 2% de gaz carbonique. Comme dans le cas précédent, les microalgues absorbent les rayonnements solaires et le carbone du gaz carbonique, et elles fabriquent des polysaccharides hydrosolubles qu'elles rejettent à travers les pores dans le milieu liquide nutritif.

Bien que ce type de réacteur permette de diminuer la dépense d'énergie nécessaire, celui-ci présente encore certains inconvénients.

En effet, les dés de polyuréthane ne sont pas très transparents et, de ce fait, le rayonnement solaire n'atteint que faiblement les dés qui se trouvent au centre du tube; d'autre part, les transferts de matières (apport de gaz carbonique et extraction des polysaccharides) se font difficilement.

La présente invention a précisément pour objet une paroi transparente en mousse de polyuréthane qui contient des microorganismes et qui peut être utilisée dans un biophotoréacteur pour pallier les inconvénients rappelés ci-dessus.

Selon l'invention, la paroi transparente est constituée par de la mousse de polyuréthane dans les pores de laquelle sont répartis des microorganismes, et elle se caractérise en ce que les pores de la mousse sont fermés sur l'une des faces de la paroi de façon telle que ladite face soit imperméable aux liquides et aux gaz alors que la face opposée de la paroi présente une porosité ouverte.

En raison des caractéristiques différentes de perméabilité des deux faces opposées de la paroi, celle-ci permet d'assurer dans de bonnes conditions la culture des microorganismes. En effet, la face imperméable de la paroi peut être exposée aux rayonnements alors que la face opposée qui présente une porosité ouverte, peut être en contact avec un milieu liquide nutritif qui pourra accéder facilement à l'intérieur de la paroi afin d'apporter aux microorganismes immobilisés dans la paroi les constituants nécessaires pour leur développement. De même, le produit synthétisé par les microorganismes peut être rejeté dans le milieu liquide nutritif en contact avec la face de la paroi qui présente une porosité ouverte.

Ainsi, on peut non seulement limiter les dépenses d'énergie comme dans le cas où l'on utilise des dés de polyuréthanne sur lesquels sont immobilisés des microorganismes, mais aussi réaliser l'irradiation par le rayonnement solaire et les transferts de matière dans de bien meilleures conditions.

Les microorganismes répartis dans les pores de la paroi peuvent être constitués par des microalgues telles que Porphyridium Cruentum et Botryococcus Braunii, ou par des bactéries, notamment par des bactéries photosynthétiques.

L'invention a également pour object un procédé de préparation d'une paroi transparente en mousse de polyuréthane contenant des microorganismes et dont l'une au moins des faces présente une porosité ouverte. Ce procédé comprend les étapes suivantes:

a) préparer un mélange liquide comprenant:

— des composés précurseurs de la mousse de polyuréthane, et

— une suspension de microorganismes dans un milieu liquide nutritif;

b) introduire ce mélange dans un moule comportant deux parois opposées, dont l'une seulement a été imprégnée d'un agent capable d'ouvrir les pores de la mousse;

c) former la mousse de matière plastique par réaction des composés précurseurs présents dans le mélange liquide; et

d) démouler la paroi ainsi obtenue.

Grâce à la présence d'un agent capable d'ouvrir les pores de la mousse sur l'une des parois du moule, on obtient après démoulage une paroi qui est poreuse sur sa face qui était en contact avec la paroi du moule imprégnée par l'agent capable d'ouvrir les pores de la mousse et qui est au contraire imperméable aux gaz et aux liquides sur son autre face qui était en contact avec l'autre paroi du moule. En effet, l'agent capable d'ouvrir les pores se dilue à la surface de la mousse en cours de formation et il fragilise les parois des pores. De ce fait, lors de l'expansion de la mousse, les parois fragilisées se déchirent et les pores s'ouvrent donc vers l'extérieur. En revanche, sur l'autre paroi du moule qui n'a pas été imprégnée par cet agent, on obtient une peau continue transparente et imperméable par concentration de matière, lors de l'expansion de la mousse.

Selon l'invention, l'agent capable d'ouvrir les pores de la mousse peut être un polyoxyalkylène à forte teneur en oxyde d'éthylène, par exemple du polyéthylène glycol, tel que PEG400, mais également un polyester polyol ou une alcanolamine.

Les composés précurseurs de la mousse de polyuréthane sont des composés capables de réagir entre eux pour former cette mousse. Généralement, les composés précurseurs sont constitués par un polyéther polyol à base d'oxyde de propylène et d'oxyde d'éthylène et par du diisocyanate de toluène.

A titre d'exemple, ces composés peuvent être constitués par des prépolymères d'uréthane commercialisés par la société TOYO RUBER AND C° qui sont constitués respectivement par un polyétherdiol ayant un poids moléculaire moyen d'environ 2600 contenant 91% d'oxyde d'éthylène et par un polyéther diol ayant un poids moléculaire moyen d'environ 2600 et une teneur en oxyde d'éthylène de 100%, la teneur en NCO des deux prépolymères étant de 4%. On peut aussi utiliser les prépolymères commercialisés sous la marque HYPOL 3000 par la Société W.R. Grace qui contiennent environ 10% en poids de diisocyanate de toluène libre ou encore des prépolymères commercialisés par Montedison ayant une teneur en NCO libre de 5%, qui nécessitent l'emploi de catalyseurs alcalins spécifiques pour la formation de la mousse.

Pour former le mélange liquide utilisé pour la préparation de la paroi, on mélange généralement une partie du prépolymère liquide précurseur de la mousse de polyuréthanne qui peut contenir jusqu'à 50% d'eau, à une partie d'une suspension de microorganismes dans un milieu liquide nutritif.

Généralement la concentration en microorganismes de la suspension est de 2 à 20 mg de matières sèches par litre et les milieux liquides nutritifs utilisés sont constitués de façon classique par les milieux utilisés habituellement pour la croissance des microorganismes. Ces milieux contiennent de nombreux constituants par exemple des composés d'azote, de phosphore, de potassium, de calcium et/ou de magnésium, des sels de fer, de zinc, de manganèse, de cuivre, de nickel, de molybdène et/ou de bore. Ils peuvent également contenir des régulateurs de croissance et éventuellement des acides aminés et des vitamines, et leurs teneurs en ces différents constituants sont choisies en fonction de la nature du microorganisme utilisé.

Selon l'invention, les parois opposées du moule peuvent être constituées, soit par des tubes concentriques, soit par des plaques planes. Ainsi, on peut obtenir la paroi transparente de l'invention soit sous la forme d'un tube dont la face qui présente une porosité ouverte constitue généralement la surface interne du tube, soit sous la forme d'une plaque dont l'une des faces est imperméable aux liquides et aux gaz alors que l'autre face est perméable aux liquides et aux gaz.

Généralement l'intervalle entre les deux parois du moule qui déterminera l'épaisseur de la paroi transparente préparée selon l'invention, est de 1 à 5 mm, et les parois du moule sont réalisées en matériau métallique, par exemple en acier.

Avant d'introduire le mélange liquide dans le moule, on imprègne l'une des parois du moule de polyoxyde d'alkylène, par exemple par application au pinceau, puis on introduit le mélange liquide et on ferme le moule. Après cette opération, la mousse se forme dans le moule à la température ambiante et la polymérisation et le durcissement de la mousse sont terminés au bout d'environ une heure. On démoule alors la paroi transparente ainsi obtenue.

L'invention a encore pour objet un biophotoréacteur pour la culture de microorganismes, qui comprend au moins une paroi transparente contenant des microorganismes et présentant les caractéristiques de perméabilité données ci-dessus, la face imperméable aux gaz et aux liquides de ladite paroi étant exposée à une source de lumière et la face opposée de ladite paroi qui présente une porosité ouverte étant en contact avec un milieu liquide nutritif et avec un gaz pour réaliser la croissance des microorganismes présents dans ladite paroi et leur permettre de synthétiser des produits désirés.

Selon un premier mode de réalisation de ce biophotoréacteur, celui-ci ne comprend qu'une seule paroi transparente et il est utilisé pour la culture d'un seul type de microorganisme. Dans ce cas, la paroi transparente a avantageusement la forme d'un tube dont la surface externe constitue la face imperméable aux gaz et aux liquides, et on met en circulation le milieu nutritif et un gaz à l'intérieur dudit tube pour réaliser la croissance des microorganismes présents dans la paroi.

Selon un second mode de réalisation du biophotoréacteur de l'invention qui est plus particulièrement adapté à la croissance de deux types de microorganismes, celui-ci comprend deux parois transparentes superposées délimitant entre elles un intervalle pour la mise en circulation d'un premier milieu liquide nutritif et d'un premier gaz; dans ce cas, la face imperméable aux gaz et aux liquides de la seconde paroi transparente qui est disposée sous la première paroi transparente, est en contact avec le premier milieu liquide nutritif circulant dans l'intervalle entre les deux parois, et la face à porosité ouverte de la seconde paroi transparente est en contact avec un second milieu liquide nutritif et un second gaz pour assurer la croissance des microorganismes disposés dans ladite seconde paroi.

Ce mode de réalisation du biophotoréacteur de l'invention est particulièrement adapté à la croissance, d'une part, de microalgues qui utilisent une partie du spectre lumineux et sont disposées dans la première paroi et, d'autre part, de bactéries photosynthétiques qui utilisent la partie complémentaire du spectre lumineux et sont disposées dans la seconde paroi.

Selon un troisième mode de réalisation du biophotoréacteur de l'invention, celui-ci comprend deux parois transparentes superposées délimitant entre elles un intervalle dans lequel on met en circulation un milieu liquide nutritif. Dans ce cas, la face à porosité ouverte de la première paroi transparente qui est située au-dessus de la seconde paroi transparente, est en contact avec le milieu liquide nutritif et la face à porosité ouverte de la seconde paroi transparente est également en contact avec le milieu liquide nutritif.

Cette disposition des deux parois est particulièrement adaptée pour réaliser la culture de microalgues capables de rejeter de l'oxygène, qui sont réparties dans les pores de la première paroi transparente, et libèrent ainsi de l'oxygène dans le milieu liquide nutritif. Dans ce cas, les microorganismes présents dans la seconde paroi transparente sont des microorganismes utilisant de l'oxygène pour leur croissance et ils peuvent ainsi consommer l'oxygène libéré dans le milieu nutritif par les microorganismes présents dans la première paroi.

D'autres caractéristiques et avantages de l'invention apparaîtront mieux à la lecture de la description qui suit donnée bien entendu à titre illustratif et non limitatif en référence au dessin annexé sur lequel:

— les figures 1 et 2 illustrent deux modes de réalisation de la paroi transparente selon l'invention,

— la figure 3 représente schématiquement la disposition de deux parois transparentes dans un biophotoréacteur, et

— la figure 4 représente un autre mode de disposition de deux parois transparentes dans un biophotoréacteur.

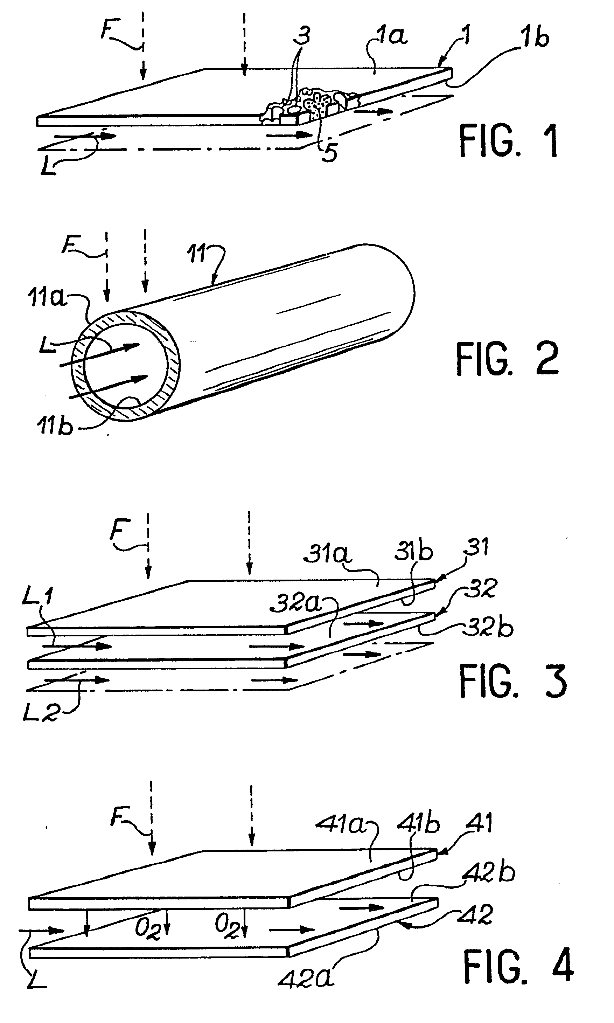

Sur la figure 1, on a représenté un premier mode de réalisation de la paroi transparente de l'invention et son mode de mise en œuvre dans un biophotoréacteur ne comprenant qu'une seule paroi transparente.

Sur cette figure, la référence 1 désigne la paroi transparente de l'invention qui est constituée par de la mousse de polyuréthanne dans les pores 3 de laquelle sont répartis des microorganismes 5. Cette paroi transparente a la forme d'une plaque plane et sa face supérieure 1a est imperméable aux gaz et aux liquides alors que sa face inférieure 1b est perméable aux gaz et aux liquides. Comme représenté sur le dessin, la face inférieure 1b est en contact avec un flux de liquide L qui s'écoule le long de la paroi pour amener, d'une part, les éléments nécessaires à la culture du microorganisme et, extraire, d'autre part, les métabolites formés par ce microorganisme, tandis que la face supérieure 1a de la paroi est exposée à un rayonnement symbolisé par les flèches F, qui peut être émis par le soleil ou par une source de lumière artificielle.

Sur la figure 2, on a représenté un autre mode de réalisation de la paroi transparente de l'invention. Dans ce cas, la paroi 11 est constituée comme précédemment d'une mousse de polyuréthanne dans les pores de laquelle sont répartis des microorganismes et elle a la forme d'un tube. La surface externe de ce tube 11a est imperméable aux gaz et aux liquides alors que la surface interne 11b est perméable aux gaz et aux liquides. De la sorte, on peut exposer la surface externe 11a à un rayonnement F et mettre en circulation à l'intérieur du tube un milieu liquide L capable d'amener, d'une part, les éléments nécessaires à la croissance des microorganismes et d'extraire, d'autre part, les produits synthétisés par ceux-ci.

Sur la figure 3, on a représenté un mode de réalisation du biophotoréacteur de l'invention dans lequel celui-ci comprend deux parois transparentes superposées. Sur cette figure, les références 31 et 32, désignent deux parois planes entre lesquelles on met en circulation un premier liquide nutritif $L_1$. La première paroi 31 est disposée à la partie supérieure du dispositif avec sa face perméable aux gaz et aux liquides 31b en contact avec le premier milieu liquide $L_1$. La seconde paroi transparente 32 est disposée sous la première paroi 31 de façon que sa face 32a imperméable aux gaz et aux liquides soit en contact avec le premier milieu liquide $L_1$. En dessous de la deuxième paroi transparente 32, on met en circulation un second milieu liquide nutritif $L_2$. Ainsi, en exposant l'ensemble à une source de lumière symbolisée par les flèches F, on peut assurer la croissance des microorganismes disposés dans la première paroi transparente 31 et recueillir dans le premier liquide $L_1$ les métabolites formés par ces microorganismes et réaliser la croissance des seconds microorganismes disposés dans la seconde paroi transparente 32 en leur apportant les éléments nécessaires et extraire du second milieu liquide $L_2$ les métabolites formés par les seconds microorganismes. Dans ce cas, les premiers microorganismes utilisent une première partie du spectre lumineux et les seconds microorganismes utilisent pour leur croissance la partie complémentaire du spectre lumineux.

A titre d'exemple, les premiers microorganismes peuvent être constitués par des microalgues telles que Porphyridium cruentum ou Botryococcus braunii, et les seconds microorganismes constitués par des bactéries photosynthétiques.

Sur la figure 4, on a représenté un autre mode de réalisation d'un biophotoréacteur comportant deux parois transparentes selon l'invention. Sur cette figure, on voit que ce biophotoréacteur comprend une première paroi transparente 41 et une seconde paroi transparente 42. La première paroi transparente 41 est disposée de façon telle que sa face 41b qui est perméable aux gaz et aux liquides soit en contact avec un milieu liquide nutritif L circulant entre les deux parois et la seconde paroi transparente 42 qui est située en dessous de la première paroi 41 est disposée de façon telle que sa face 42b perméable aux gaz et aux liquides soit en contact également avec le milieu liquide nutritif L. Ainsi, par irradiation au moyen d'une source lumineuse de la partie supérieure 41a du photoréacteur, on réalise la croissance des microorganismes présents dans la première paroi transparente 41. Ces microorganismes libèrent dans le milieu liquide nutritif L des éléments qui peuvent être utilisés pour la croissance des microorganismes présents dans la seconde paroi transparente 42 et l'on extrait du milieu liquide nutritif les produits synthétisés par les premiers et/ou les seconds microorganismes.

A titre d'exemple, la première paroi 41 peut comprendre des microalgues capables de rejeter de l'oxygène dans le milieu liquide et cet oxygène peut être utilisé par des microorganismes présents dans la seconde paroi transparente 42.

L'exemple suivant de réalisation d'une paroi transparente est donné à titre l'illustration de l'invention.

Dans cet exemple, on utilise comme composé précurseur de la mousse de polyuréthanne, un prépolymère de polyétherpolyol à base d'oxyde de propylène (80%) et d'oxyde d'éthylène (20%) et de toluène diisocyanate tel qu'on ait un excès de groupes -N = C = 0 de 1,45 et un rapport -NCO/OH égal à 1,87, on ajoute au prépolymère 50% d'une suspension de microorganismes Porphyridium cruentum obtenue par dilution dans 500 ml de milieu de Hemerick de 37 g d'une pâte de microorganisme à 90% d'eau obtenue par culture dans les conditions suivantes: 25°C; milieu minéral liquide de Hemerick à pH 6,9; barbotage avec de l'air à 5% de $CO_2$ à raison de 10 volumes par volume de culture et par heure.

On introduit ce mélange dans un moule plat en acier dont le fond a été imprégné de polyéthylène glycol PEG400 de poids moléculaire «400» par application au pinceau.

Au bout d'environ une heure à la température ambiante, on démoule la paroi obtenue. La face de cette paroi qui était en contact avec le PEG400 présente une porosité ouverte alors que les autres faces de la paroi sont imperméables aux gaz et aux liquides.

On utilise cette paroi comme paroi supérieure d'un biophotoréacteur et on expose sa face imperméable à une source de lumière telle que l'énergie reçue sur cette surface est de 28 $Wm^{-2}$, soit 578 $kcal.m^{-2}j^{-1}$ et on fait circuler sur l'autre face le milieu minéral de Hemerick à pH 6,9 et de l'air contenant 2% de $CO_2$. On extrait du milieu sortant du bioréacteur les polysaccharides sulfatés formés par la méthode de Ramus.

## Revendications

1. Paroi transparente constituée par de la mousse de polyuréthane dans les pores (3) de laquelle sont répartis des microorganismes (5), caractérisée en ce que les pores de la mousse sont fermés sur l'une des faces (1a) de la paroi de façon telle que ladite face soit imperméable aux liquides et aux gaz alors que la face opposée (1b) de la paroi présente une porosité ouverte.

2. Paroi selon la revendication 1, caractérisée en ce que les microorganismes sont des algues.

3. Paroi selon la revendication 1, caractérisée en ce que les microorganismes sont des bactéries photosynthétiques.

4. Procédé de préparation d'une paroi transparente selon l'une quelconque des revendications 1 à 3, caractérisé en ce qu'il comprend les étapes suivantes:

a) préparer un mélange liquide comprenant:
— des composés précurseurs de la mousse de polyuréthane, et
— une suspension de microorganismes dans un milieu liquide nutritif;

b) introduire ce mélange dans un moule comportant deux parois opposées dont l'une seulement a été imprégnée d'un agent capable d'ouvrir les pores de la mousse;

c) former la mousse de matière plastique par réaction des composés précurseurs présents dans le mélange liquide; et

d) démouler la paroi transparente ainsi obtenue.

5. Procédé selon la revendication 4, caractérisé en ce que l'agent capable d'ouvrir les pores de la mousse est un polyoxyalkylène à forte teneur en oxyde d'éthylène, un polyester polyol ou une alcanolamine.

6. Procédé selon la revendication 5, caractérisé en ce que le polyoxyalkylène est du polyéthylène glycol.

7. Procédé selon l'une quelconque des revendications 4 à 6, caractérisé en ce que les composés précurseurs de la mousse de polyuréthane sont un polyéther polyol à base d'oxyde de propylène et d'oxyde d'éthylène et du diisocyanate de toluène.

8. Procédé selon l'une quelconque des revendications 4 à 7, caractérisé en ce que les parois opposées du moule sont constituées par des tubes concentriques.

9. Procédé selon l'une quelconque des revendications 4 à 7, caractérisé en ce que les parois opposées du moule sont des plaques planes.

10. Biophotoréacteur pour la culture de microorganismes, caractérisé en ce qu'il comprend au moins une paroi transparente (1) selon l'une quelconque des revendications 1 à 3, la face imperméable (1a) aux gaz et aux liquides de ladite paroi étant exposée à une source de lumière F et la face (1b) de ladite paroi qui présente une porosité ouverte étant en contact avec un milieu liquide nutritif (L) et avec un gaz pour réaliser la croissance des microorganismes présents dans ladite paroi et leur permettre de synthétiser des produits désirés.

11. Biophotoréacteur selon la revendication 10, caractérisé en ce que la paroi transparente (11) a la forme d'un tube dont la surface externe (11a) constitue la face imperméable aux gaz et aux liquides, et en ce que l'on met en circulation le milieu nutritif et un gaz à l'intérieur dudit tube.

12. Biophotoréacteur selon la revendication 10, caractérisé en ce qu'il comprend deux parois transparentes superposées (31, 32) délimitant entre elles un intervalle pour la mise en circulation d'un premier milieu liquide nutritif (L₁) et d'un premier gaz, en ce que la face imperméable (32a) aux gaz et aux liquides de la seconde paroi transparente qui est disposée sous la première paroi transparente, et en contact avec le premier milieu liquide nutritif (L₁) circulant dans l'intervalle entre les deux parois, et en ce que la face à porosité ouverte (32b) de la seconde paroi transparente est en contact avec un second milieu liquide nutritif (L₂) et un second gaz pour assurer la croissance des microorganismes disposés dans ladite seconde paroi.

13. Biophotoréacteur selon la revendication 12, caractérisé en ce que la première paroi (31) contient des microalgues et la seconde paroi (32) contient des bactéries photosynthétiques.

14. Biophotoréacteur selon la revendication 10, caractérisé en ce qu'il comprend deux parois transparentes superposées (41, 42) délimitant entre elles un intervalle dans lequel on met en circulation un milieu liquide nutritif (L), la face à porosité ouverte (41b) de la première paroi transparente qui est située au-dessus de la seconde paroi transparente étant en contact avec le milieu liquide nutritif (L), et la face à porosité ouverte (42b) de la seconde paroi transparente étant également en contact avec le milieu liquide nutritif.

15. Biophotoréacteur selon la revendication 14, caractérisé en ce que la première paroi (41) contient des microalgues capables de produire et de rejeter de l'oxygène dans le milieu liquide nutritif et en ce que la seconde paroi (42) contient des microorganismes utilisant de l'oxygène pour leur croissance.

**Patentansprüche**

1. Transparente Wand aus Polyurethanschaum, in dessen Poren (3) Mikroorganismen (5) verteilt sind, dadurch gekennzeichnet, dass die Poren des Schaums auf einer der Oberflächen (1a) der Wand geschlossen sind, so dass diese Oberfläche für Flüssigkeiten und Gase undurchlässig ist, während die gegenüberliegende Oberfläche (1b) der Wand eine offene Porosität aufweist.

2. Wand nach Anspruch 1, dadurch gekennzeichnet, dass es sich bei den Mikroorganismen um Algen handelt.

3. Wand nach Anspruch 1, dadurch gekennzeichnet, dass es sich bei den Mikroorganismen um Photosynthese-Bakterien handelt.

4. Verfahren zur Herstellung einer transparenten Wand nach einem der Ansprüche 1 bis 3, dadurch gekennzeichnet, dass es die folgenden Stufen umfasst:

a) die Herstellung einer flüssigen Mischung, die umfasst:

— Vorläuferverbindungen des Polyurethanschaums und

— eine Suspension von Mikroorganismen in einem flüssigen Nährmilieu;

b) die Einführung dieser Mischung in eine Form mit zwei einander gegenüberliegenden Wänden, von denen nur eine mit einem Agens imprägniert worden ist, das die Poren des Schaums öffnen kann;

c) die Bildung des Kunststoffschaums durch Umsetzung der in der flüssigen Mischung vorhandenen Vorläuferverbindungen; und

d) die Entformung der so erhaltenen transparenten Wand.

5. Verfahren nach Anspruch 4, dadurch gekennzeichnet, dass es sich bei dem Agens, das die Poren des Schaums öffnen kann, um ein Polyoxyalkylen mit hohem Ethylenoxidgehalt, ein Polyesterpolyol oder ein Alkanolamin handelt.

6. Verfahren nach Anspruch 5, dadurch gekennzeichnet, dass es sich bei dem Polyoxyalkylen um Polyethylenglycol handelt.

7. Verfahren nach einem der Ansprüche 4 bis 6, dadurch gekennzeichnet, dass es sich bei den Vorläuferverbindungen für den Polyurethanschaum um ein Polyätherpolyol auf Basis von Propylenoxid und Ethylenoxid und Toluoldiisocyanat handelt.

8. Verfahren nach einem der Ansprüche 4 bis 7, dadurch gekennzeichnet, dass die einander gegenüberliegenden Wände der Form aus konzentrischen Rohren gebildet sind.

9. Verfahren nach einem der Ansprüche 4 bis 7, dadurch gekennzeichnet, dass die einander gegenüberliegenden Wände der Form ebene Platten sind.

10. Biophotoreaktor zur Kultivierung von Mikroorganismen, dadurch gekennzeichnet, dass er umfasst mindestens eine transparente Wand (1) nach einem der Ansprüche 1 bis 3, deren für Gase und Flüssigkeiten undurchlässige Oberfläche (1a) einer Lichtquelle F ausgesetzt ist und deren Oberfläche (1b), die eine offene Porosität aufweist, mit einem flüssigen Nährmilieu (L) und mit einem Gas in Kontakt steht, um das Wachstum der in dieser Wand vorhandenen Mikroorganismen zu bewirken und die Synthese der gewünschten Produkte durch sie zu erlauben.

11. Biophotoreaktor nach Anspruch 10, dadurch gekennzeichnet, dass die transparente Wand (11) die Form eines Rohres hat, dessen äussere Oberfläche (11a) die für Gase und Flüssigkeiten undurchlässige Oberflächen darstellt, und dass man das Nährmilieu und ein Gas im Innern des Rohres zirkulieren lässt.

12. Biophotoreaktor nach Anspruch 10, dadurch gekennzeichnet, dass er zwei übereinanderliegende transparente Wände (31, 32) umfasst, die einen dazwischenliegenden Zwischenraum begrenzen, in dem ein erstes flüssiges Nährmedium (L₁) und ein erstes Gas zirkulieren können, so dass die für Gase und Flüssigkeiten undurchlässige Oberfläche (32a) der zweiten transparenten Wand, die unter der ersten transparenten Wand angeordnet ist, in Kontakt steht mit dem ersten flüssigen Nährmilieu (L$_1$), das in dem Zwischenraum zwischen den beiden Wänden zirkuliert, und dass die Oberfläche mit einer offenen Porosität (32b) der zweiten transparenten Wand mit einem zweiten flüssigen Nährmilieu (L$_2$) und einem zweiten Gas in Kontakt steht, um das Wachstum der in dieser zweiten Wand angeordneten Mikroorganismen zu gewährleisten.

13. Biophotoreaktor nach Anspruch 12, dadurch gekennzeichnet, dass die erste Wand (31) Mikroalgen enthält und dass die zweite Wand (32) Photosynthese-Bakterien enthält.

14. Biophotoreaktor nach Anspruch 10, dadurch gekennzeichnet, dass er zwei übereinanderliegende transparente Wände (41, 42) umfasst, die einen dazwischenliegenden Zwischenraum begrenzen, in den man ein flüssiges Nährmilieu (L) zirkulieren lässt, wobei die Oberfläche mit einer offenen Porosität (41b) der ersten transparenten Wand, die oberhalb der zweiten transparenten Wand angeordnet ist, mit dem flüssigen Nährmilieu (L) in Kontakt steht und die Oberfläche mit einer offenen Porosität (42b) der zweiten transparenten Wand ebenfalls mit dem flüssigen Nährmilieu in Kontakt steht.

15. Biophotoreaktor nach Anspruch 14, dadurch gekennzeichnet, dass die erste Wand (41) Mikroalgen enthält, die Sauerstoff in dem flüssigen Nährmilieu bilden und abgeben können, und dass die zweite Wand (42) Mikroorganismen enthält, die Sauerstoff für ihr Wachstum ausnutzen.

## Claims

1. Transparent wall consisting of polyurethane foam, in the pores (3) of which microorganisms (5) are distributed, characterized in that the pores of the foam are closed on one of the faces (1a) of the wall so that the said face is impermeable to liquids and gases, whereas the opposite face (1b) of the wall possesses open porosity.

2. Wall according to Claim 1, characterized in that the microorganisms are algae.

3. Wall according to Claim 1, characterized in that the microorganisms are photosynthetic bacteria.

4. Process for preparing a transparent wall according to any one of Claims 1 to 3, characterized in that it comprises the following stages:

a) Preparing a liquid mixture comprising:

precursor compounds of the polyurethane foam, and a suspension of microorganisms in a nutrient liquid medium;

b) Introducing this mixture into a mould containing two opposite walls, only one of which has been impregnated with an agent capable of opening the pores of the foam;

c) Forming the plastic foam by reaction of the precursor compounds present in the liquid mixture; and

d) removing the transparent wall thereby obtained from the mould.

5. Process according to Claim 4, characterized in that the agent capable of opening the pores of the foam is a polyoxyalkylene having a high content of ethylene oxide, a polyesterpolyol or an alkanolamine.

6. Process according to Claim 5, characterized in that the polyoxyalkylene is polyethylene glycol.

7. Process according to any one of Claims 4 to 6, characterized in that the precursor compounds of the polyurethane foam are a polyetherpolyol based on propylene oxide and ethylene oxide, and toluene diisocyanate.

8. Process according to any one of Claims 4 to 7, characterized in that the opposite walls of the mould consist of concentric tubes.

9. Process according to any one of Claims 4 to 7, characterized in that the opposite walls of the mould are flat plates.

10. Biophotoreactor for culturing microorganisms, characterized in that it comprises at least one transparent wall (1) according to any one of Claims 1 to 3, the face (1a) which is impermeable to gases and liquids of the said wall being exposed to a light source F, and the face (1b) of the said wall which possesses open porosity being in contact with a nutrient liquid medium (L) and with a gas to effect the growth of the microorganisms present in the said wall and enable them to synthesize desired products.

11. Biophotoreactor according to Claim 10, characterized in that the transparent wall (11) is in the form of a tube whose outer surface (11a) constitutes the face which is impermeable to gases and liquids, and in that the nutrient medium and a gas are passed inside the said tube.

12. Biophotoreactor according to Claim 10, characterized in that it comprises two superposed transparent walls (31, 32) which between them demarcate a space for the passage of a first nutrient liquid medium (L$_1$) and a first gas, in that the face (32a) which is impermeable to gases and liquids of the second transparent wall, which is arranged under the first transparent wall, is in contact with the first nutrient liquid medium (L$_1$) passing in the space between the two walls, and in that the face (32b) having open porosity of the second transparent wall is in contact with a second nutrient liquid medium (L$_2$) and a second gas to provide for the growth of the microrganisms arranged in the said second wall.

13. Biophotoreactor according to Claim 12, characterized in that the first wall (31) contains microalgae and the second wall (32) contains photosynthetic bacteria.

14. Biophotoreactor according to Claim 10, characterized in that it comprises two superposed transparent walls (41, 42) which between them demarcate a space in which a nutrient liquid medium (L) is passed, the face (41b) having open porosity of the first transparent wall, which is situated above the second transparent wall, being in contact with the nutrient liquid medium (L), and the face (42b) having open porosity of the second transparent wall also being in contact with the nutrient liquid medium.

15. Biophotoreactor according to Claim 14, characterized in that the first wall (41) contains microalgae capable of producing and discharging oxygen into the nutrient liquid medium and in that the second wall (42) contains microorganisms which use the oxygen for their growth.

FIG. 1

FIG. 2

FIG. 3

FIG. 4